Europäisches Patentamt

European Patent Office

Office européen des brevets

(19)

(11) Publication number: **0 336 736 B1**

# EUROPEAN PATENT SPECIFICATION

(12)

(45) Date of publication of patent specification: 04.05.94  (51) Int. Cl.5: **A61K 39/10, C07K 3/18**

(21) Application number: 89303369.6

(22) Date of filing: 05.04.89

The file contains technical information submitted after the application was filed and not included in this specification

(54) **Purification of pertussis toxins and production of vaccine.**

(30) Priority: 05.04.88 GB 8807860

(43) Date of publication of application:
11.10.89 Bulletin 89/41

(45) Publication of the grant of the patent:
04.05.94 Bulletin 94/18

(84) Designated Contracting States:
AT BE CH DE ES FR GB GR IT LI LU NL SE

(56) References cited:
EP-A- 0 121 249
EP-A- 0 175 841
EP-A- 0 252 838

INFECTION AND IMMUNITY, vol. 41, no. 1, July 1983, American Society for Microbiology; Y. SATO et al., pp. 313-320&NUM;

VACCINE, vol. 3, no. 1, March 1985, Butterworth & CO. (publishers/ Ltd., Guildford, Surrey, GB; A. ROBINSON et al., pp. 11-22&NUM;

CHAMBERS' DICTIONARY OF SCIENCE AND TECHNOLOGY, 1971; p. 871&NUM;

(73) Proprietor: **CONNAUGHT LABORATORIES LIMITED**
**1755 Steeles Avenue West**
**Willowdale Ontario M2R 3T4(CA)**

(72) Inventor: **Tan, Larry U. L.**
**2424 Folkway Drive**
**Missisauga, ON, L5L 3N3(CA)**
Inventor: **Jackson, Gail**
**87 Springhead Gardens**
**Richmond Hill, ON, L4C 5C3(CA)**
Inventor: **Alkema, Dirk**
**224 Simcoe Street**
**Stayner, ON, L0M 1S0(CA)**
Inventor: **Wah, Po S.**
**7 Parravano Court**
**Willowdale, ON, M2R 3S8(CA)**
Inventor: **Fahim, Rafaat Emil Fahmy**
**524 Ceremonial Drive**
**Missisauga, ON, L5R 3n3(CA)**

(74) Representative: **Burford, Anthony Frederick et al**
**W.H. Beck, Greener & Co.**
**7 Stone Buildings**
**Lincoln's Inn**
**London WC2A 3SZ (GB)**

EP 0 336 736 B1

COLLLINS' DICTIONARY OF THE ENGLISH
LANGUAGE, 1979; p. 1092&NUM;

## Description

The present invention relates to a novel method of isolation and purification of specific proteins from the fermentation broth of the organism Bordetella pertussis, the removal of pyrogenic factors from these proteins, the detoxification of the proteins and the preparation from these purified proteins of a vaccine that is virtually non-toxic, has little or no side effects and confers protection against the disease of pertussis.

For infants below the age of 24 months, the disease caused by Bordetella pertussis, pertussis or whooping cough, can be very severe and has a mortality rate of approximately 1%. Over the last fifty years, three types of vaccine have been available for immunization against the disease. The most-widely used vaccine, that has been available for the protection of infants against pertussis infection, in combination with tetanus and diphtheria vaccines, is the so-called "whole cell" vaccine, which is available in all developed countries, and many of the Third World countries.

This whole cell pertussis vaccine is prepared by growing known strains of the B.pertussis organism in a defined medium for several hours in a fermentor, until the mixture reaches certain defined parameters. The mixture then is treated with a chemical agent, such as formaldehyde, which kills the organism and detoxifies proteins present in the supernatant and in the organism itself. After allowing the mixture to stand for a specified time to ensure that this detoxification procedure is complete, the cells are separated from the supernatant by passing the mixture through a continuous centrifuge, to provide a packed mass of cells and the supernatant, which is discarded. The cells then are resuspended in a solution of sodium chloride to provide a suspension, that, when diluted to a known strength, usually determined by the opacity of the suspension, and injected subcutaneously, elicits antibodies that are protective against the disease. This "whole cell" vaccine is known for giving minor local reactions at the injection site with occasionally more severe overall reactions, such as elevated temperature and general fretfulness. There has been speculation that the vaccine is responsible for some neurological reactions in infants.

In a second vaccine type, the B.pertussis was grown and detoxified with formaldehyde as before, and then the isolated cells extracted with a concentrated solution of urea. After filtration and dialysis to remove the urea, a mixture of soluble cell wall components is obtained, which, after dilution to a known strength, was in use as a vaccine from 1969 to 1974, but has now been withdrawn because of poor efficacy. More recently, a third vaccine type, commonly called an acellular vaccine, that is in use in Japan and is in clinical trials in a number of other developed countries, is prepared by isolation and purification of constituents of the culture supernatants of B.pertussis after detoxification. Specifically, the constituents called lymphocytosis promoting factor (LPF) also known as pertussis toxin (PT), filamentous hemaglutinin (FHA) and agglutinogens have been isolated and identified. However, because of variations in growth of the organism and the method of isolation, which is non-specific, the composition of the isolated mixture of proteins can vary. At present there is no vaccine in general use and licensed that is completely non-toxic and still gives good protection.

With the growth of the science of immunology over the years, it has been recognized that protective antibodies against a specific disease can be elicited by the administration of specific cellular components of the organism that causes the disease, rather than the whole organism that has been inactivated or has been attenuated to give a non-pathogenic strain. It has been recognized that use of detoxified or attenuated organisms as a vaccine can introduce components that may be damaging to the recipient. With this in mind, a number of efforts have been made to isolate components of the pertussis organism, either from the cell or that have been excreted into the medium, that could have antigenic capabilities, be completely non-toxic and thus could act as vaccines.

As yet there has been no convincing proof that any one particular cell component, by itself, can act as a protective antigen. However, in a number of publications and Patent applications (see, for instance, EP-A-0231083 and EP-A-0175841) it has been suggested that a mixture of the purified and detoxified proteins, lymphocytosis promoting factor (LPF) and filamentous hemaglutinin (FHA), can act as a combined antigen and, when administered to a mammal, generate antibodies that confer protection against the disease. In the aforesaid publications various methods have been disclosed for obtaining these proteins in a purified form, and once purified using them in varying proportions as a vaccine.

EP-A-0175841 discloses the purification of LPF and FHA separately from a B. pertussis fermentation broth by contacting a LPF- or FHA- containing solution with a cellulose sulphate gel, a polysaccharide gel chemically bound with dextran sulphate or a crosslinked polysaccharide sulphate gel and eluting the adsorbed FHA or LPF from the gel. The solutions are usually regulated to a conductivity of 0.5 to 5.0 mS/cm (LPF) or 5.0 to 25.0 mS/cm (FHA) and the elute usually is a buffer of conductivity 5 to 100 mS/cm (LPF) or 25 to 130 mS/cm (FHA).

3

The separation and purification of LPF and FHA from culture supernatants of B. pertussis using hydroxylapatite, haptoglobin-Sepharose™ and Sepharose CL-6B™ filtration chromatography has been described by Y. Sato et al (Infect. Immun. 41 (1983) 313-320).

EP-A-0121249 discloses the detoxification of a fraction containing LPF and FHA by a treatment with formalin in the presence of an amino acid. The presence of the amino acid is reported to substantially reduce the detoxification treatment time and to avoid aggregate precipitation.

Although existing technology produces highly purified LPF and FHA, the processes have inherent drawbacks. The affinity chromatographic methods that have been used are effective but conditions of adsorption and elution often use materials which are toxic, expensive and/or denature the required proteins. In addition, some of the materials used on affinity columns can be leached into the product under the harsh elution conditions required and, since some of the leached materials are blood derived, may introduce the possibility of blood born diseases or autosensitization. The use of gel filtration materials and hydroxyapatite are acceptable when used by themselves, but give only low purification factors.

The LPF and FHA, which are produced by B.pertussis, also represent a major challenge in the removal of lipopolysaccharides (LPS) as a contaminant. LPS even in nanogram quantities can produce fever and are an undesirable component of any vaccine. The initial concentration of LPS in the fermentation supernatant can be as high as one milligram per millilitre. A number of methods have been used to remove pyrogens from vaccines (see, for instance, US-A-4,000,257 and US-A-4,380,511). Many of these methods are too harsh and result in denaturation of the required proteins. Other methods are ineffective, cumbersome to use and expensive.

Before using the LPF and FHA in a vaccine, these proteins must be detoxified since the LPF in its natural state is highly toxic and small amounts are still present in the purified FHA. This process previously has been achieved by treating the proteins with a chemical agent which induces cross-linking. Traditionally, the agents used have been formaldehyde and glutaraldehyde. The use of formaldehyde and glutaraldehyde can lead to heavy losses due to aggregation and precipitation.

In accordance with the present invention, there is provided a novel method for the isolation and purification of the proteinaceous materials LPF and FHA from the growth medium of B.pertussis by adsorption and desorption on various substrates, using a combination of low and high ionic strength solutions. In addition, there is provided an improved method of removing pyrogenic factors, as exemplified by LPS, from the LPF and FHA by washing the adsorbed proteins with a detergent solution. Further, there is provided an improved method for the detoxification of the LPF and FHA, using a cross-linking agent in the presence of an anti-aggregation agent, such that the purified materials can be readily combined into an efficacious vaccine for the prevention of the disease of pertussis.

Accordingly, in one aspect of the present invention, there is provided a method for the isolation and purification of the proteinaceous materials called lymphocytosis promoting factor (LPF) and filamentous hemaglutinin (FHA) from a growth medium in which has been grown the Bordetella pertussis organism. The method comprises contacting the growth medium at low ionic strength with a non-derivatized solid particulate adsorbing medium to selectively adsorb LPF and FHA from the growth medium, and desorbing the proteinaceous materials by contacting the adsorbing medium with an aqueous medium of high ionic strength.

The isolated LPF and FHA, after further purification and detoxification, can be formulated into a non-toxic vaccine for protection against pertussis.

The inventors have determined that LPF and FHA can be adsorbed preferentially from the filtered growth medium of B.pertussis, at low ionic strength, onto a variety of solid particulate adsorbent materials. After the LPF and FHA are adsorbed from the growth supernatant at low ionic strength onto the substrates, they are desorbed from the adsorbent material using an aqueous solution of high ionic strength.

The high ionic strength desorbing medium is an aqueous solution of a salt and/or buffer. The term "salt solution" used herein refers to all metal or ammonium salts, such as potassium nitrate, sodium chloride and ammonium sulfate, which when dissolved in water, dissociate into their constituent ions, thereby increasing the ionic strength of the solution without significantly changing the pH of the solution. The term "buffer" used herein refers to a chemical compound which, when dissolved in water, dissociates into their constituent ions, thereby increasing the ionic strength of the solution and having buffering capacity.

As used herein, the term "low ionic strength", refers to an aqueous medium having a conductivity of 11 mS/cm or less, preferably about 4 mS/cm. The unit of measurement mS/cm is millisiemen per centimeter. A siemen (S) is a unit of conductivity and is the equivalent of the inverse of resistance (ohm) and is sometimes designated mho. The term "high ionic strength" as used herein refers to an aqueous medium having a conductivity of greater than 11 mS/cm and preferably at least 50 mS/cm.

Solid particulate adsorbent materials useful in the present invention include filter aids, such as perlite (which is of volcanic ash origin) and Celite™ (a diatomaceous earth believed to be a solid solution of dicalcium silicate in dicalcium aluminate), siliceous materials, such as sand, celluloses, agaroses and gel filtration materials, such as the Sepharoses™, the Sephadexes™, Ultragel™ and their derivatives.

The variety of matrix materials which have been found useful for the adsorbing medium in the present invention suggests that the characteristics of the matrix material are non-critical but rather it is the property of LPF and FHA that, under low ionic strength conditions, they will bind to a large variety of matrices.

While not wishing to be bound by any particular theory to explain the process of the invention, it is thought that, under the initial low ionic strength conditions employed, the LPF and FHA are close to coming out of solution. By passing the solution in contact with insoluble particulate matrices, the particles of the matrix act as nuclei onto which the LPF and FHA can precipitate. Resolubilization for desorbtion then requires a higher ionic strength solution.

After desorption from the adsorbing medium by the high ionic strength solution, a mixture of the two proteins is obtained, that can be further separated on other materials, such as hydroxyapatite or other ion-exchange resins, to give the two proteins in high yields and high purity. Alternatively, we have found that separation of FHA and LPF after adsorption onto the adsorbing medium can be obtained by desorbing from the adsorbing medium at differing ionic strengths. To effect preferential elution of LPF from the adsorbing medium, an ionic strength of solution of 11 mS/cm to 20 mS/cm is employed. Once the LPF has been eluted, FHA can be eluted at an ionic strength of solution of at least 20 mS/cm, preferably at least 50 mS/cm.

The ability to effect adsorption at low ionic strength and subsequent elution at high ionic strength of LPF and FHA on conventional gel filtration media and the other non-derivatized adsorbing media used herein is totally unexpected and deviates from the state of the art, where proteins are not adsorbed to gel filtration media and where the protein is continuously eluted from the column under isocratic, i.e., a single buffer, conditions. The gel media have been chosen in previous work because of their very low non-specific protein adsorption, and yet, under the conditions of the invention, they will still adsorb the LPF and FHA very well. It has been shown by the inventors that FHA and LPF can be purified to a greater degree on agarose than derivatized agarose.

The method can be used either as a batch process on the cell free media obtained from the growth of the organism or as a separation method on a chromatography column of the adsorbent. Because of their ease of filtration, their low costs and the accepted employment of filter-aids in the manufacture of pharmaceutical products, the use of the filter-aids is preferable to the use of other materials, such as gel filtration media.

The inventors have further found that, if the proteins adsorbed onto the matrices are washed, before elution, with an aqueous non-ionic detergent solution, the LPS in the final product can be reduced by a factor of 10,000 to 100,000, to a concentration of 1 to 10 ng/mL. Examples of suitable non-ionic detergent solutions are Triton X-100™ in a concentration of 0.005 to 5% (v/v), preferably 0.1 to 1% (v/v), and Nonidet p40™ in a concentration of 0.0005 to 0.1% (v/v), preferably 0.001 to 0.01% (v/v). Triton X-100™ is believed to be an alkylphenol alkoxylate and Nonidet p40™ is believed to be an alkylene oxide condensate.

It has also been found by the inventors that the purified LPF and FHA can be detoxified by contact with a cross-linking agent, such as glutaraldehyde and/or formaldehyde in the presence of an anti-aggregation agent, such as glycerol or sucrose, to improve the yield of final product. The anti-aggregation agent is present in a significant proportion during the detoxification operation and prevents the aggregation and precipitation that occurs in the absence of such material. For the detoxification of LPF in the presence of glycerol, glycerol is present in an amount of 30 to 80% (v/v), preferably approximately 50%, while for the detoxification of FHA, glycerol is present in an amount of 10 to 80% (v/v), preferably approximately 25%. Where sucrose is used as the anti-aggregation agent, the sucrose is present in an amount of 30 to 60% (w/v), preferably approximately 40%.

In the present invention, B.pertussis is grown in a fermentor using controlled conditions. Carbon sources and growth factors are supplemented continuously or in batches at various intervals during the fermentation until the two proteins, LPF and FHA, are at the desired level, which can be determined by enzyme linked immunosorbent assay (ELISA). In the invention, the mixture of cells and medium from the fermentor is not inactivated by chemical means immediately after the fermentation is complete, but later in the purification process. The use of chemical detoxification at this stage in the process can lead to aggregation of the proteins and poor separation in the following steps.

The fermentor then is harvested and the majority of the cells removed by continuous centrifugation. The remainder of the cells then can be removed from the supernatant by filtration, using known membrane filters of 0.2μ pore size, which also sterilizes the solution. In the present invention, it is this supernatant that

5

is retained and processed for isolation and purification of LPF and FHA. After centrifugation and filtration, the supernatant is concentrated, say 10-fold, using membrane filtration, assayed for protein and then diluted until the ionic strength is in the required range. This solution, which contains the LPF and FHA proteins then is treated with the adsorbing medium, either in a batch process or as a chromatographic process. The LPF and FHA, after adsorption onto the adsorbing medium, are washed first with several volumes of a buffer to remove contaminants and then with a solution of a detergent which removes the majority of the lipopolysaccharides (LPS). Further washing removes any traces of the detergent.

The LPF and FHA can be obtained separately from the adsorbent by elution with solutions of stepwise increasing ionic strength or the two proteins can be eluted together using a high ionic strength salt solution. The proteins can be further purified on a chromatography column of a material, such as hydroxyapatite. The proteins are eluted from such a column by using different ionic strengths solutions after prior washing. The separate proteins then are detoxified in the presence of an anti-aggregation agent to result in high yields of detoxified protein. After removal of the additives, the detoxified proteins are sterilized by filtration and, after assay, can be mixed in the required proportion to give a solution that can be used as a vaccine against pertussis.

The procedure of the invention may be employed to effect large scale separation of LPF and FHA using a chromatographic column of perlite, which is currently the best mode known to the applicants for effecting separation and recovery of purified LPF and FHA.

Concentrated B.pertussis fermentation broth is diluted to a low ionic strength corresponding to a conductivity of 4mS or less and loaded onto a column of packed perlite to provide a protein loading of 1 to 5 mg, preferably 2 to 3 mg per millilitre of packed perlite. The packed perlite column usually is 15 to 18 cm high and 10 to 45 cm in diameter.

The dilute fermentation broth is contacted with the perlite column at a linear flow rate of 50 to 200 cm/hr, preferably approximately 100 cm/hr. The proteins which are adsorbed to the perlite are almost exclusively LPF and FHA with most of the contaminating proteins and LPS passing through the column.

The column then is washed with 2 to 10 column volumes of a buffer containing 10 to 50 mM, of Tris HCl at pH 8.0. A subsequent wash with an aqueous non-ionic detergent, typically about 5 column volumes of an 0.5% (v/v) Triton X-100™ solution in 50 mM Tris HCl buffer at pH 8.0, decreases the LPS content of the proteins by a factor of about 100, for a total decrease in the LPS/LPF ratio of between 10,000 and 100,000. Subsequent washing of the column with further volumes preferably about 5 volumes of buffer, of 50 mM Tris HCl at pH 8.0, removes the non-ionic detergent.

The LPF is eluted from the column by contacting the column with buffer solution, for example, 5 volumes of 50 mM Tris HCl at pH 8.0, containing 0.1 to 0.2 M sodium chloride, preferably about 0.12 M. The FHA next is eluted from the column by contacting the column with buffer solution, for example, 5 volumes of 50 mM Tris HCl at pH 8.0, containing at least 0.2 M of sodium chloride, preferably about 0.6 M.

The eluted solutions are assayed for protein content. By this procedure, LPF and FHA recoveries of approximately 60 to 65% and 65 to 70% respectively of the initial contents of these proteins in the broth have been obtained.

Further purification of LPF and FHA may be effected using a column of packed hydroxyapatite 5 to 8 cm in height and 5 to 30 cm in diameter. The column is washed and equilibrated prior to use. The eluate containing LPF is applied to the column at a loading of 0.5 to 1 mg/ml of packed gel at a linear flow rate of 15 to 25 cm/hr to adsorb the LPF therefrom.

The column is washed with a suitable buffer, for example, 5 column volumes of 30 mM potassium phosphate at pH 8.0, following which the LPF is eluted with 5 to 10 column volumes of an eluting medium, for example, 75 mM potassium phosphate at pH 8.0, containing 0.1 to 0.3 M sodium chloride, preferably about 0.225 M.

The procedure may be repeated for the FHA-containing eluant, with elution being effected using an aqueous elution medium, for example, 200 mM potassium phosphate at pH 8.0, containing at least 0.2 M sodium chloride, preferably about 0.6 M.

In these hydroxyapatite purification procedures, typical recoveries of the pure protein are 80 to 100% with the respective proteins having a purity of at least 90%.

Detoxification of the further purified LPF and FHA may be effected in order to provide these materials in a form suitable for formulation as a non-toxic vaccine. It is preferred to effect detoxification of the LPF protein using glutaraldehyde in the presence of glycerol while it is preferred to effect detoxification of the FHA protein using formaldehyde in the presence of glycerol.

The invention is illustrated further by the following Examples.

## EXAMPLES

Methods of protein biochemistry, fermentation and assays used but not explicitly described in this disclosure and these Examples are amply reported in the scientific literature and are well within those skilled in the art.

## Example 1

This Example illustrates the growth of B.pertussis in fermentors.

Bordetella pertussis was seeded into a fermentor containing 250 L of broth (modified Stainer-Scholte medium). During the period of fermentation, monosodium glutamate (2.18 kg) and the growth factors, glutathione (41 g), ferrous sulphate (2.7 g), calcium chloride (5.5 g), ascorbic acid (109 g), niacin (1.1 g) and cysteine (10.9 g), were added at intervals to increase the yields of LPF. At the end of a 48 hour fermentation period, the broth was run through a continuous centrifuge to remove the majority of the cells. This suspension, which contains both the LPF and FHA in solution, was further clarified by micro-filtration on cellulose acetate membranes (0.22 $\mu$m pore size). The sterilized filtrate was concentrated approximately 10-fold using a 20,000 NML membrane and then assayed for protein by the dye-binding method.

## Example 2

This Example illustrates the isolation of LPF and FHA on a number of different matrices.

A number of 1 millilitre columns were packed with various matrices and equilibrated with 50 mM Tris HCl at pH 8.0, 10 mM potassium phosphate at pH 8.0 or water. The matrices included Orange A-, Blue A-, Green A-, Red A-agaroses*, Blue Sepharose™*, Blue B-, Reactive Blue 4-, Cibacron™ Blue 3GA-, Reactive Brown 10-, Reactive Green 19-, Reactive Yellow 86-Sepharose™*, non-derivatized agarose, Ultragel ACA44™, Sephadex G50™, Sepharose 6B™, Sepharose CL4B™, S-Sepharose™*, Q-Sepharose™*, cellulose sulphate*, QAE-cellulose*, CM-cellulose*, perlite and Celite™.

B. pertussis culture broth was centrifuged, sterile filtered through a 0.2 $\mu$ membrane and concentrated approximately 10 fold by ultrafiltration on 20 kD NML membranes. Broth concentrates were diluted with water so that the ionic strength was less than or equal to 4 mS/cm. Samples between 2 to 10 ml were loaded onto the columns by gravity feed and then washed with excess 10 mM potassium phosphate, followed by 50 mM Tris HCl buffer at pH 8.0. Each column was eluted with 50 mM Tris HCl at pH 8.0 containing either 0.6 M or 1.0 M sodium chloride. Fractions were analysed by absorbance at 280 nm and on SDS-PAGE. All of the matrices were found to adsorb LPF and FHA. The eluted LPF and FHA were found to be highly purified.

In a similar experiment using white quartz sand, a column 1.5 cm in diameter and 18 cm in height was washed and loaded with the same diluted broth concentrate to adsorb LPF and FHA therefrom and washed. The column then was eluted first with 50 mM Tris HCl at pH 8.0 containing 0.1 M sodium chloride, followed by Tris buffer containing 1.0 M sodium chloride, so as to elute first the LPF and then the FHA. The separately eluted LPF and FHA respectively were found to be highly purified.

## Example 3

This Example illustrates the large scale separation of LPF and FHA using a chromatographic column of perlite.

The broth concentrate, prepared as described in Example 1, was diluted with water to a conductivity of approximately 4 mS/cm, such that the final loading of protein was approximately 3 mg of crude protein per millilitre of packed perlite. The packed perlite column was 18 cm high and 10 cm in diameter and was prewashed with 1.4 L of Water for Injection (WFI). The diluted concentrate was applied to the column at a linear flow rate of 100 cm/hr. The proteins bound to the perlite were almost exclusively LPF and FHA with most of the contaminating protein and lipopolysaccharide (LPS) passing through. The column was washed with 1.4 L of a buffer containing 50 mM Tris HCl at pH 8.0. A subsequent wash with detergent, composed of 1.4 L of a 0.5% (v/v) Triton X-100™ solution in 50 mM Tris HCl buffer at pH 8.0, reduced the LPS content by a further factor of 100, for a total reduction in the LPS/LPF ratio of between 10,000 to 100,000. The column then was washed with a further 1.4 L 50 mM Tris HCl at pH 8.0 to remove the Triton X-100™. The LPF then was eluted from the column with 50 mM Tris HCl at pH 8.0 containing 0.12 M sodium chloride.

(* derivatized material)

The FHA was eluted from the column using 50 mM Tris HCl at pH 8.0 containing 0.6 M sodium chloride. Approximately 1.4 L of each elution buffer was used. The solutions then were assayed for protein content by the dye-binding assay. LPF and FHA recoveries were 60% and 65%, respectively, based on ELISA values.

### Example 4

This Example illustrates the batch adsorption of LPF and FHA on perlite.

B.pertussis broth concentrates (60 ml) were diluted 4-fold with water to a conductivity of approximately 4 mS/cm and perlite (2g) added. The mixture was rotated slowly at 4°C for 3 hr. The mixture was vacuum filtered on a sintered glass filter and the residual perlite was rinsed into the filter with 50 mM Tris HCl at pH 8.0 (20 ml). The perlite was washed with 4 x 50 ml of the Tris buffer and then eluted with 3 x 20 ml of 50 mM Tris HCl at pH 8.0 containing 1.0 M sodium chloride. The eluates were pooled and assayed using an ELISA assay. LPF recoveries were calculated to be at least 65%.

### Example 5

This Example illustrates the further purification of LPF on hydroxyapatite.

Hydroxyapatite was packed into a column 5 to 30 cm diameter and 6 cm height. The column was washed with 200 mM potassium phosphate at pH 8.0, 1 M potassium chloride, 0.5% Triton X-100™ and equilibrated with 10 mM potassium phosphate at pH 8.0 prior to use. The LPF solution, recovered as described in Example 3, was applied to the column at a loading of approximately 0.5 mg of protein/ml of packed gel at a linear flow rate of approximately 20 cm/hr. The column was washed with 500 ml of 30 mM potassium phosphate at pH 8.0. The LPF was eluted with 1 L of 75 mM potassium phosphate at pH 8.0 containing 0.225 M sodium chloride. The resulting LPF was at least 90% pure. The LPF was assayed for protein by the dye binding method. The LPF recovery was approximately 90% for this step.

### Example 6

This Example illustrates the further purification of FHA on hydroxyapatite.

The hydroxyapatite was packed and washed in a column of the same size as detailed in Example 5. The FHA fraction from the perlite separation described in Example 3 was applied to the column at a linear flow rate of 20 cm/hr and a loading of 0.5 mg of protein/ml of packed gel. The column was washed with 500 ml each of 30 mM potassium phosphate at pH 8.0, 30 mM potassium phosphate at pH 8.0 containing 0.5% (v/v) of Triton X-100™ and 30 mM potassium phosphate at pH 8.0. Any remaining LPF in the fraction first was eluted with 500 ml of 85 mM potassium phosphate at pH 8.0 and the FHA then was eluted with 200 mM potassium phosphate at pH 8.0 containing 0.6 M potassium chloride. The resulting FHA was at least 90% pure. The FHA was assayed for protein by the Lowry method. FHA recovery for this column was approximately 90%.

### Example 7

This Example illustrates the detoxification of LPF with glutaraldehyde.

The purified LPF, prepared as described in Example 5, in 75 mM potassium phosphate at pH 8.0 containing 0.22 M sodium chloride was diluted with an equal volume of glycerol to a protein concentration of approximately 200 $\mu$g/ml. The solution was heated to 37°C and detoxified by the addition of glutaraldehyde to a final concentration of 0.5% (w/v). The mixture was kept at 37°C for 4 hr and followed by the addition of aspartic acid (1.5 M) to a concentration of 0.25 M. The mixture was incubated at room temperature for 1 and then diafiltered with 10 volumes of 10 mM potassium phosphate at pH 8.0 containing 0.15 M sodium chloride to remove both the glycerol and the glutaraldehyde. The LPF toxoid was sterile filtered through a 0.2 $\mu$ membrane.

### Example 8

This Example illustrates the detoxification of FHA with formaldehyde.

The purified FHA, prepared as described in Example 6, in 200 mM potassium phosphate at pH 8.0 containing 0.6 M potassium chloride was diluted with glycerol to give a final concentration of 25% V/V. The protein concentrations was approximately 500 $\mu$g/ml based on the Lowry protein assay. The FHA solution was heated to 37°C and a 1.5 M solution of L-lysine HCl at pH 8.0 was added to a final concentration of 50 mM. Formaldehyde was added to a final concentration of 0.25% V/V. Detoxification was carried out at 37°C for a period of 6 weeks. The resulting toxoid was diafiltered against 10 volumes of 10 mM potassium phosphate at pH 8.0 containing 0.5 M sodium chloride to remove both the glycerol and the formaldehyde. The toxoid solution was sterile filtered through a 0.2$\mu$ membrane.

## Example 9

This Example illustrates the use of detoxified LPF and FHA in producing protective antibodies.

Guinea pigs (SPF) were prescreened for pertussis antibody titres, and only those animals which showed low background titres were used in the experiment.

Animals were injected with 0.5 ml of test material at day zero. Test materials employed in the tests were adsorbed and unadsorbed purified and detoxified LPF and FHA products produced by the procedures of Examples 7 and 8 respectively and conventional whole cell vaccine.

Four weeks after injection, the animals were bled and the sera tested for PT and FHA antibodies by ELISA. Sera also were tested for CHO antitoxin activity. At day 35, the animals were boosted with the same dose of antigen and finally the animals were bled at day 49 and the sera tested. The results are shown in the following Table I:

**TABLE I**

**IMMUNOGENICITY OF PERTUSSIS TOXOID**

**In guinea pigs at 25 ug dose**

| | Protein $\mu$g | CHO Units 1st 2nd | ELISA X $10^{-3}$ | | | |
|---|---|---|---|---|---|---|
| | | | LPF | | FHA | |
| | | | 1st bleed | 2nd bleed | 1st bleed | 2nd bleed |
| LPF (unadsorbed) | 25 | 14      640 | 3 | 256 | | |
| (adsorbed) | 25 | 433    1664 | 59 | 410 | | |
| FHA (unadsorbed) | 25 | | | | 52 | 33 |
| (adsorbed) | 25 | | | | 14 | 205 |
| Whole Cell (unadsorbed) | human dose | 4        30 | 2 | 21 | 4 | 21 |

**All results are reciprocal reactive titres.**

The results set forth in the Table indicate that when compared to the conventional whole cell vaccine, both adsorbed and unadsorbed purified and detoxified LPF and FHA proteins provided by the procedures of the invention give considerably higher antibody titres.

## Example 10

This Example illustrates the use of the purified antigens in the mouse protection test.

Taconic mice (15 to 17g) were injected at day zero with 0.5 ml of the test sample intraperitoneally, in three doses. Each dose was injected into 16 mice. At day 14, the mice were challenged with an intracerebral injection of a standard dose of B.pertussis. Control mice also were injected at the same time to ascertain the effectiveness of the challenge. Three days after the challenge, the number of animal deaths was recorded every day up to and including day 28. At day 28, paralysed mice and mice with cerebral edema also were recorded as dead.

Results were recorded as $ED_{50}$, which is the dose at which half the mice survive the challenge. This was done using a computer programme after plotting the survivors divided by the total number of mice in each category at each dose.

The result of this experiment showed that the $ED_{50}$ of a mixture of LPF and FHA was less than [1$\mu$g LPF + 2$\mu$g FHA], and thus a mixture of the two purified proteins was protective against the disease.

9

**Claims**

1.  A method for the isolation and purification of the proteinaceous materials called lymphocytosis promoting factor (LPF) and filamentous hemaglutinin (FHA) from a growth medium in which has been grown the <u>Bordetella pertussis</u> organism, wherein (a) the growth medium, diluted if necessary, of conductivity of 11 mS/cm or less is contacted with a non-derivatized solid particulate adsorbing medium to selectively adsorb LPF and FHA from the growth medium, and (b) the adsorbed proteinaceous materials are desorbed by contacting said adsorbing medium with an aqueous medium of conductivity greater than 11 mS/cm and sufficient to effect said desorption.

2.  A method as claimed in Claim 1, wherein the adsorbing medium is a filter aid, a siliceous material, a cellulose, an agarose or gel filtration medium.

3.  A method as claimed in Claim 2, wherein the adsorbing medium is a perlite of volcanic ash origin.

4.  A method as claimed in Claim 2, wherein the adsorbing medium is a diatomaceous earth which is a solid solution of dicalcium silicate in dicalcium aluminate.

5.  A method as claimed in any one of the preceding claims, wherein the (diluted) growth medium has conductivity of 4 mS/cm or less when contacting the adsorbing medium.

6.  A method as claimed in any one of the preceding claims, wherein the FHA and LPF are desorbed from the adsorbing medium in a single desorbing operation.

7.  A method as claimed in Claim 6, wherein the aqueous medium has an conductivity at least 50 mS/cm when contacting the adsorbing medium to effect the desorption.

8.  A method as claimed in any one of Claims 1 to 5, wherein the adsorbing medium first is contacted with an aqueous medium having conductivity sufficient to selectively desorb LPF in preference to FHA and then is contacted with an aqueous medium having conductivity sufficient to desorb FHA.

9.  A method as claimed in Claim 8, wherein the first-mentioned aqueous medium has conductivity from 11 mS/cm to 20 mS/cm and the second-mentioned aqueous medium has conductivity of at least 20 mS/cm.

10. A method as claimed in any one of the preceding claims, wherein subsequent to the adsorption step and prior to the desorption step, said absorbing medium is contacted with an aqueous non-ionic detergent solution selective to elute lipopolysaccharides (LPS) from the adsorbing medium in preference to LPF and FHA.

11. A method as claimed in Claim 10, wherein the non-ionic detergent solution is effective to decrease the LPS contamination of said LPF and FHA to 1 to 10 ng/ml.

12. A method as claimed in Claim 10 or Claim 11, wherein the non-ionic detergent is an alkylphenol alkoxylate or an alkylene oxide condensate.

13. A method as claimed in any one of the preceding claims, wherein subsequent to the desorption step, the desorbed LPF and FHA are subjected to further purification using hydroxyapatite.

14. A method as claimed in any one of the preceding claims, wherein subsequent to the desorption step, the purified proteinaceous material is detoxified by contact with a cross-linking agent in the presence of an anti-aggregation agent.

15. A method as claimed in Claim 14, wherein the anti-aggregation agent is glycerol or sucrose.

16. A method as claimed in Claim 15, wherein the anti-aggregation agent is glycerol present in an amount of 30 to 80% (v/v) for detoxification of LPF or in an amount of 10 to 80% (v/v) for detoxification of FHA.

**17.** A method of preparing a vaccine against pertussis, which comprises preparing a detoxified proteinaceous material by a method as claimed in any one of Claims 1 to 13, and sterilizing and formulating as a vaccine said detoxified proteinaceous material.

**18.** A method of preparing a vaccine against pertussis, which comprises preparing a detoxified proteinaceous material by a method as claimed in any one of Claims 14 to 16, and sterilizing and formulating as a vaccine said detoxified proteinaceous material.

**Patentansprüche**

**1.** Verfahren zur Isolierung und Reinigung proteinartiger Materialien, die Lymphocytose-fördernder Faktor (LPF) und filamentöses Hämaglutinin (FHA) genannt werden, aus einem Wachstumsmedium, in dem der Organismus <u>Bordetella</u> <u>pertussis</u> gezogen wurde, worin (a) das Wachstumsmedium, falls nötig verdünnt, mit einer Leitfähigkeit von 11 mS/cm oder weniger mit einem underivatisierten, festen, partikulären Adsorptionsmedium zusammengebracht wird, um LPF und FHA aus dem Wachstumsmedium selektiv zu adsorbieren, und (b) die adsorbierten proteinartigen Materialien durch Zusammenbringen dieses Adsorptionsmediums mit einem wässrigen Medium, das eine Leitfähigkeit größer als 11 mS/cm aufweist und zur Bewirkung dieser Desorption ausreicht, desorbiert werden.

**2.** Verfahren nach Anspruch 1, worin das Adsorptionsmedium eine Filterhilfe, ein kieselartiges Material, eine Cellulose, eine Agarose oder ein Gelfiltrationsmedium ist.

**3.** Verfahren nach Anspruch 2, worin das Adsorptionsmedium ein Perlit ist, der aus vulkanischer Asche stammt.

**4.** Verfahren nach Anspruch 2, worin das Adsorptionsmedium eine Diatomeenerde ist, die eine feste Lösung aus Dicalciumsilicat in Dicalciumaluminat darstellt.

**5.** Verfahren nach einem der vorangehenden Ansprüche, worin das (verdünnte) Wachstumsmedium eine Leitfähigkeit von 4 mS/cm oder weniger aufweist, wenn es mit dem Adsorptionsmedium zusammengebracht wird.

**6.** Verfahren nach einem der vorangehenden Ansprüche, worin FHA und LPF in einem einzigen Desorptionsschritt vom Adsorptionsmedium desorbiert werden.

**7.** Verfahren nach Anspruch 6, worin das wässrige Medium eine Leitfähigkeit von mindestens 50 mS/cm aufweist, wenn es mit dem Adsorptionsmedium zusammengebracht wird, um die Desorption zu bewirken.

**8.** Verfahren nach einem der Ansprüche 1 bis 5, worin das Adsorptionsmedium zuerst mit einem wässrigen Medium zusammengebracht wird, das eine zur selektiven Desorption von LPF vor FHA ausreichende Leitfähigkeit aufweist und dann mit einem wässrigen Medium zusammengebracht wird, das eine zur Desorption von FHA ausreichende Leitfähigkeit aufweist.

**9.** Verfahren nach Anspruch 8, worin das zuerst erwähnte wässrige Medium eine Leitfähigkeit von 11 mS/cm bis 20 mS/cm aufweist und das als zweites erwähnte wässrige Medium eine Leitfähigkeit von mindestens 20 mS/cm aufweist.

**10.** Verfahren nach einem der vorangehenden Ansprüche, worin nach dem Adsorptionsschritt und vor dem Desorptionsschritt dieses Adsorptionsmedium mit einer wässrigen nicht-ionischen Detergenzlösung zusammengebracht wird, die für die Elution von Lipopolysacchariden (LPS) vor LPF und FHA vom Adsorptionsmedium selektiv ist.

**11.** Verfahren nach Anspruch 10, worin die nicht-ionische Detergenzlösung zur Herabsetzung der LPS Kontamination von LPF und FHA auf 1 bis 10 ng/ml wirksam ist.

**12.** Verfahren nach Anspruch 10 oder 11, worin das nicht-ionische Detergenz ein Alkylphenolalkoxylat oder ein Alkylenoxidkondensat ist.

**13.** Verfahren nach einem der vorangehenden Ansprüche, worin anschließend an den Desorptionsschritt das desorbierte LPF und FHA einer weiteren Reinigung mittels Hydroxyapatit unterzogen werden.

**14.** Verfahren nach einem der vorangehenden Ansprüche, worin anschließend an den Desorptionsschritt das gereinigte proteinartige Material durch Zusammenbringen mit einem quervernetzenden Mittel in Gegenwart eines Antiaggregationsmittels entgiftet wird.

**15.** Verfahren nach Anspruch 14, worin das Antiaggregationsmittel Glycerin oder Saccharose ist.

**16.** Verfahren nach Anspruch 15, worin das Antiaggregationsmittel Glycerin ist, das zur Entgiftung von LPF in einer Menge von 30 bis 80 % (V/V) oder zur Entgiftung von FHA in einer Menge von 10 bis 80 % (V/V) vorkommt.

**17.** Verfahren zur Herstellung eines Impfstoffes gegen Pertussis, das die Herstellung eines entgifteten proteinartigen Materials mittels eines Verfahrens nach einem der Ansprüche 1 bis 13 und die Sterilisierung und Formulierung dieses entgifteten proteinartigen Materials als Impfstoff umfaßt.

**18.** Verfahren zur Herstellung eines Impfstoffes gegen Pertussis, das die Herstellung eines entgifteten proteinartigen Materials mittels eines Verfahrens nach einem der Ansprüche 14 bis 16 und die Sterilisierung und Formulierung dieses entgifteten proteinartigen Materials als Impfstoff umfaßt.

**Revendications**

**1.** Méthode pour isoler et purifier les matières protéiniques dites facteur favorisant la lymphocytose (LPF) et hémaglutinine filamenteuse (FHA) à partir d'un milieu de croissance dans lequel a été cultivé l'organisme *Bordetella pertussis*, dans laquelle (a) le milieu de croissance, dilué si nécessaire, de conductivité égale ou inférieure à 11 mS/cm est mis au contact d'un milieu adsorbant à particules solides non dérivatisé pour que la LPF et la FHA du milieu de culture soient adsorbés sélectivement, et (b) les matières protéiniques adsorbées sont désorbées par la mise en contact dudit milieu adsorbant avec un milieu aqueux de conductivité supérieure à 11 mS/cm et suffisant pour produire ladite désorption.

**2.** Méthode selon la revendication 1, dans laquelle le milieu adsorbant est un adjuvant de filtration, une matière siliceuse, une cellulose, un agarose, ou un milieu de filtration de gel.

**3.** Méthode selon la revendication 2, dans laquelle le milieu adsorbant est une Perlite provenant de cendres volcaniques.

**4.** Méthode selon la revendication 2, dans laquelle le milieu adsorbant est une terre à diatomées qui est une solution solide de silicate dicalcique dans de l'aluminate dicalcique.

**5.** Méthode selon une quelconque des revendications qui précèdent, dans laquelle le milieu de croissance (dilué) a une conductivité égale ou inférieure à 4 mS/cm quand il est au contact du milieu adsorbant.

**6.** Méthode selon une quelconque des revendications qui précèdent, dans laquelle les FHA et LPF sont désorbés du milieu adsorbant dans une opération de désorption unique.

**7.** Méthode selon la revendication 4, dans laquelle le milieu aqueux a une conductivité d'au moins 50 mS/cm quand il est mis en contact avec le milieu adsorbant pour produire une désorption.

**8.** Méthode selon une quelconque des revendications 1 à 5, dans laquelle le milieu adsorbant est d'abord mis au contact d'un milieu aqueux ayant une conductivité suffisante pour désorber le LPF sélectivement par rapport à la FHA puis est mis au contact d'un milieu aqueux ayant une conductivité suffisante pour désorber la FHA.

**9.** Méthode selon la revendication 8, dans laquelle le premier milieu aqueux mentionné a une conductivité allant de 11 mS/cm à 20 mS/cm et le second milieu aqueux mentionné a une conductivité d'au moins 20 mS/cm.

10. Méthode selon une quelconque des revendications qui précèdent, dans laquelle après l'étape d'adsorption et avant l'étape de désorption, ledit milieu adsorbant est mis au contact d'une solution aqueuse de détergent non ionique sélective pour éluer les lipopolysaccharides (LPS) du milieu adsorbant de préférence par rapport aux LPF et FHA.

11. Méthode selon la revendication 10, dans laquelle la solution de détergent non ionique est efficace pour diminuer la contamination desdits LPF et FHA par les LPS jusqu'à 1 à 10 ng/ml.

12. Méthode selon la revendication 10 ou la revendication 11, dans laquelle le détergent non ionique est un alcoxylate d'alkylphénol ou un condensat d'oxyde d'alkylène.

13. Méthode selon une quelconque des revendications qui précèdent, dans laquelle, après l'étape de désorption, les LPF et FHA désorbés sont soumis à une repurification à l'aide d'hydroxyapatite.

14. Méthode selon une quelconque des revendications qui précèdent, dans laquelle après l'étape de désorption, le matériau protéinique purifié est détoxifié par contact avec un agent réticulant en présence d'un agent anti-agrégation.

15. Méthode selon la revendication 14, dans laquelle l'agent anti-agrégation est le glycérol ou le saccharose.

16. Méthode selon la revendication 15, dans laquelle l'agent anti-agrégation est du glycérol présent dans une quantité de 30 à 80 % (V/V) pour la détoxification du LPF ou dans une quantité de 10 à 80 % (V/V) pour la détoxification de la FHA.

17. Méthode de préparation d'un vaccin contre la coqueluche, qui comprend la préparation d'un matériau protéinique détoxifié par une méthode selon une quelconque des revendications 1 à 13, et la stérilisation et la formulation sous forme de vaccin dudit matériau protéinique détoxifié.

18. Méthode de préparation d'un vaccin contre la coqueluche, qui comprend la préparation d'un matériau protéinique détoxifié par une méthode selon une quelconque des revendications 14 à 16, et la stérilisation et la formulation sous forme de vaccin dudit matériau protéinique détoxifié.